# EUROPEAN PATENT APPLICATION

(11) **EP 3 358 069 A1**
(43) Date of publication of application: **08.08.2018**
(21) Application number: 16850386.0
(22) Date of filing: 29.09.2016
(51) Int. Cl.: D06F 58/10

(54) **PURIFICATION DEVICE**

(30) Priority: 01.10.2015 JP 2015196028
(71) Applicant: Qingdao Haier Washing Machine Co., Ltd., Shandong 266101 (CN); Aqua Co., Ltd., Chiyoda-ku Tokyo 100-0005 (JP)
(72) Inventor: OE, Katsumi, Tokyo 100-0005 (JP); KITAKAWA, Hiroyuki, Tokyo 100-0005 (JP); SANO, Masakazu, Tokyo 100-0005 (JP); FUKUI, Toshimichi, Tokyo 100-0005 (JP)
(74) Representative: Pfenning, Meinig & Partner mbB
(86) International application number: PCT/CN2016/100894
(87) International publication number: WO 2017/054762

(57) **Abstract**

Disclosed is a purification device (1), including a case part (2) having an internal space (3) for accommodating an article; and a purification unit (4) disposed in the internal space (3). The purification unit (4) includes: a flow path (41) provided with a flow inlet (51) and a flow outlet (52) which are in communication with the internal space (3); a fan (46) enabling air to circulate between the flow path (41) and the internal space (3); an ozone generating device (43) generating ozone from air; a cover (40) covering the ozone generating device (43); a supply line (44) taking air from the internal space (3) through an intake port (44C) and supplying the air to the ozone generating device (43); and a guide-in line (45) guiding the ozone generated by the ozone generating device (43) into the flow path (41). The intake port (44C) is exposed outside of the cover (40) to the internal space (3).

## Description

### TECHNICAL FIELD

The present invention relates to a purification device for purifying an accommodated article through ozone.

### BACKGROUND

In a fixed suit-deodorization dryer described in the following patent document 1, a drying chamber for accommodating clothes and a machine chamber disposed under the drying chamber are arranged within a main body which is formed into a shape of a case. The interior of the machine chamber is provided with a blower, a heater arranged in a pipeline connected to a blowing outlet of the blower, an air pump and an ozone generating device connected to an exhaust tube of the air pump. The pipeline is connected to a pipeline chamber which becomes wider towards the drying chamber. Air guided into the machine chamber from an air suction port of a front surface of the machine chamber is fed into the pipeline by the blower and is heated into hot air when passing through the heater. In addition, the ozone generating device produces ozone from the air fed through the air pump. The ozone is supplied into the pipeline by the exhaust tube and mixed with the hot air. The hot air and the ozone are supplied into the drying chamber from a bottom thereof via the pipeline chamber. Thus, the clothes in the drying chamber are dried through the hot air and are deodorized through the ozone.

Under a condition that articles such as the clothes are purified through the ozone like the suit-deodorization dryer described in the patent document 1, the purification performance can be improved with rise of the concentration of the ozone.

### Related Technical Document

### Patent Document

Patent Document 1: Japanese patent publication No. 2006-149821

### SUMMARY

### Problems to be solved by the invention

Based on the background, the present disclosure aims to provide a purification device capable of increasing ozone concentration and realizing improvement of purification performance.

### Solution for solving the problems

A purification device of the present invention includes: a case part having an internal space for accommodating an article; and a purification unit disposed in the internal space and used to purify the article in the internal space, wherein the purification unit includes: a flow path having a flow inlet and a flow outlet which are in communication with the internal space and enabling air to flow from the flow inlet to the flow outlet; a fan having a rotation blade disposed on the flow path, wherein the rotation of the rotation blade enables the air in the internal space to flow in the flow path from the flow inlet and to flow out to the internal space from the flow outlet, so that the air circulates between the flow path and the internal space; an ozone generating device generating ozone from air; a cover at least covering the ozone generating device; a supply line having an intake port exposed outside of the cover to the internal space, taking the air from the internal space through the intake port and supplying the air to the ozone generating device; and a guide-in line connecting the ozone generating device and the flow path and guiding the ozone generated by the ozone generating device into the flow path.

In addition, in the present invention, the guide-in line is connected to a region of the flow path closer to a side of the flow outlet than the rotation blade.

In addition, in the present invention, the purification device includes at least one of a handle and truckles arranged on the case part.

In addition, in the present invention, the purification device is provided with a plurality of flow outlets; and the purification device includes a hose connected to the flow outlets and having flexibility and stretchability.

### Effects of the invention

According to the present invention, for the purification unit disposed in the internal space of the case part in the purification device that includes the case part, through the rotation of the rotation blade disposed on the flow path having the flow inlet and the flow outlet, the air in the internal space flows in the flow path from the flow inlet and flows out to the internal space from the flow outlet. Thus, an air flow that circulates between the flow path and the internal space is produced. In the purification unit, the air from the internal space is taken into the supply line through the intake port of the supply line and is supplied to the ozone generating device; the ozone generating device generates the ozone from the air; the ozone generated by the ozone generating device is guided into the flow path by the guide-in line; and the ozone is poured to the article in the internal space along with the air that flows in the flow path. Thus, the article is purified. Specifically, the article is deodorized or sterilized.

Since the intake port of the supply line is exposed from the internal space outside the cover that covers the ozone generating device, the ozone is exposed in the air flow that circulates between the flow path and the internal space. Thus, since the supply line can promote air supply to the ozone generating device through forcibly taking the air in the internal space from the intake port, the ozone generating device can produce more ozone. Therefore, the purification device can increase the ozone concentration in the internal space and can realize improvement of purification performance.

In addition, according to the present invention, since the guide-in line is connected to a region of the flow path closer to a side of the flow outlet than the rotation blade, the ozone guided into the flow path by the guide-in line can flow out to the internal space from the flow outlet without being dispersed by the rotating rotation blade. Therefore, the purification device can increase the ozone concentration in the internal space and can realize further improvement of the purification performance.

In addition, according to the present invention, the purification device can be moved by holding the handle arranged on the case part. In addition, the truckles arranged on the case part are rotated on ground, so that the purification device can be smoothly moved.

In addition, according to the present invention, a plurality of flow outlets are provided. Therefore, since the ozone is poured on a plurality of articles from the respective flow outlets under a condition that the plurality of articles are accommodated in the internal space, the articles can be simultaneously purified.

When the articles cannot be configured in positions of the flow outlets of the internal space, the hose connected to the flow outlets is bent or stretched, so that the top end of the hose faces the articles and the ozone from the flow outlets is directly poured on the articles from the top end of the hose.

### BRIEF DESCRIPTION OF DRAWINGS

FIG. 1 is a perspective diagram illustrating a purification device under a vertical state according to an embodiment of the present invention;
FIG. 2 is a perspective diagram illustrating a purification device which is put down and opened;
FIG. 3 is a perspective diagram illustrating a state that a cover of a purification unit in FIG. 2 is removed;
FIG. 4 is a perspective diagram illustrating an ozone generating device;
FIG. 5 is a perspective diagram illustrating a purification unit;
FIG. 6 is a curve chart illustrating a relationship between a supply line connected to an ozone generating device and a time-dependent change of an ozone concentration produced by the ozone generating device;
FIG. 7 is a curve chart illustrating a relationship between a guide-in line connected to an ozone generating device and a time-dependent change of an ozone concentration produced by the ozone generating device; and
FIG. 8 is a perspective diagram illustrating a purification device which is put down and opened and in which an article is placed.

### List of reference numerals

1: purification device 2: case part 3: internal space 4: purification unit 5: handle 6: truckle 40: cover 41: flow path 43: ozone generating device 44: supply line 44C: intake port 45: guide-in line 46: fan 51: flow inlet 52: flow outlet 71: rotation blade 75: hose B: region P: article

### DETAILED DESCRIPTION

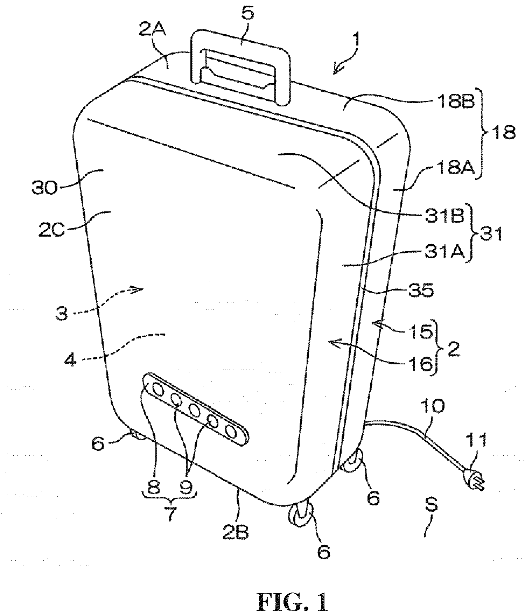

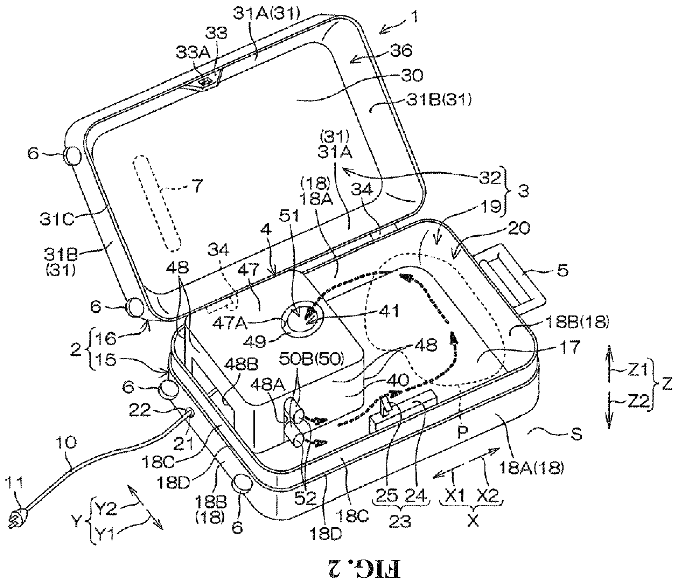

Embodiments of the present invention are described in detail below with reference to drawings. FIG. 1 is a perspective diagram illustrating a purification device 1 under a vertical state according to an embodiment of the present invention. The purification device 1 is used for deodorizing and sterilizing (purifying) an article P (see FIG. 2 below), such as shoes, a bag, a cap, a rag doll and a pillow which cannot be washed with a washing machine, through ozone. The purification device 1 includes a hollow case part 2 forming a housing of the purification device 1 and a purification unit 4 configured in an internal space 3 of the case part 2.

As shown in FIG. 1, under a state that the purification device 1 is vertical, the case part 2 is formed into, for example, a lengthwise substantial cuboid shape and has an upper surface 2A and a lower surface 2B which are substantially rectangular. The upper surface 2A is provided with a handle 5, and four corners of the lower surface 2B each are provided with a truckle 6. The handle 5 may be fixed in a posture of being always orthogonal with the upper surface 2A, and may also be connected to the upper surface 2A in a manner of turning along the upper surface 2A when not in use. The purification device 1 can be moved by holding the handle 5. At this moment, the truckles 6 arranged on the case part 2 are turned on the ground S so that the purification device 1 can be moved smoothly. Thus, the purification device 1 can be moved to various places such as indoor living rooms, hallways, balconies, closets, and can be used in a moving destination. Namely, the purification device 1 can be moved to a place requiring the purification device 1 as required. It should be noted that the respective quantity of the handle 5 and the quantity of the truckles 6 may be changed optionally. In addition, one of the handle 5 and the truckles 6 can be removed as required.

An operating part 7 is arranged on one side surface 2C of the case part 2 that connects the upper surface 2A and the lower surface 2B. The operating part 7 includes a base part 8 which is longer in a transverse direction in FIG. 1 and a plurality of buttons 9 configured on a surface of the base part 8 side by side. The user can operate the buttons 9 to start or stop the purification operation of the purification device 1 or to set operation conditions of the purification operation. A wire 10 pulled out of the case part 2 and an electrical plug 11 connected to a top end of the wire 10 are shown in FIG. 1. The case part 2 can be divided into a first case body 15 and a second case body 16 in positions in which four truckles 6 are divided into pairs of two.

FIG. 2 is a perspective diagram illustrating a purification device 1 which is put down and opened. In FIG. 2, the first case body 15 is in a state of lying on the ground S, and the second case body 16 is in a state of being suspended in the air over the ground S. The first case body 15 and the second case body 16 are formed to have a disk shape with the similar shape and size.

The first case body 15 integrally includes a substantially-rectangular bottom plate 17 and four side plates 18 erected from four sides of the bottom plate 17 towards a same direction. In FIG. 2, the bottom plate 17 is in a state of being along the ground S. In FIG. 2, a direction orthogonal with the ground S is called as an up-down direction Z; a long-edge direction of the substantially-rectangular bottom plate 17 is called as a left-right direction X; and a short-edge direction of the bottom plate 17 is called as a front-rear direction Y. The up-down direction Z includes a direction away from the ground S (an upper side Z1) and a direction close to the ground S (a lower side Z2). The left-right direction X includes a left side X1 equivalent to a left side in FIG. 2 and a right side X2 equivalent to a right side in FIG. 2. The front-rear direction Y includes a front side Y1 equivalent to a lower side in FIG. 2 and a rear side Y2 equivalent to an upper side in FIG. 2. In the following illustration, the directions are used to define orientations, postures and the like of members of the purification device 1.

In the four side plates 18, two side plates 18 erected from a pair of long edges of the bottom plate 17 are called as long side plates 18A, and two side plates 18 erected from a pair of short edges of the bottom plate 17 are called as short side plates 18B. The long side plates 18A are longer than the short side plates 18B. In the two short side plates 18B, the short side plate 18B on the left side X1 is constructed between left ends of a pair of long side plates 18A, and the short side plate 18B on the right side X2 is constructed between right ends of a pair of long side plates 18A. Connecting parts between the bottom plate 17 and the side plates 18 and connecting parts between the side plates 18 can be in a shape of rounded corners as shown in FIG. 2, or a sharp shape with a substantially right angle.

The first case body 15 has a concave space 19 which is partitioned by the bottom plate 17 and the four side plates 18 and which is concave towards the bottom plate 17. The concave space 19 is exposed from a substantially-rectangular opening 20 partitioned by upper edges of the four side plates 18. For the four side plates 18, the plate thickness of respective upper end parts 18C located on the side of the opening 20 is smaller than the plate thickness of part located on the lower side Z2 of the upper end parts 18, and a stepped surface 18D is formed at a boundary between the upper end parts 18C and the part closer to the lower side Z2 than the upper end part 18C. Respective upper end parts 18C of the four side plates 18 are located in positions closer to the side of the concave space 19 than the stepped surface 18D. The stepped surface 18D of the four side plates 18 is continuous in a coplanar state, and is integrally formed into a frame shape that encircles the opening 20.

On the left side surface of the short side plate 18B on the left side X1, two truckles 6 are arranged in a manner that the truckles 6 are away from each other in the front-rear direction Y. A through hole 21 through the short side plate 18B in the left-right direction X is formed in a region between the two truckles 6 of the short side plate 18B on the left side X1. The through hole 21 is used for the wire 10 to penetrate therethrough. A clearance between the wire 10 and a circumferential part of the through hole 21 is filled with annular fillers 22. The handle 5 is arranged on the right side surface of the short side plate 18B on the right side X2.

An unlocking mechanism 23 is arranged on a rear side surface of the long side plate 18A on the front side Y1. The unlocking mechanism 23 includes a case-shaped body part 24 with a built-in actuating mechanism (not shown) composed of a solenoid and the like, and a locking part 25 which is protruded from the body part 24 towards the upper side Z1. The body part 24 is fixed to the rear side surface of the long side plate 18A on the front side Y1. An upper end part of the locking part 25 is formed into a hook shape that is bent towards the front side Y1. The locking part 25 can be turned forwards and backwards centering on a lower end part of the locking 25. The locking part 25 in FIG. 2 is located in a locking position. The locking part 25 can be turned to an unlocking position closer to the rear side Y2 than the locking position or be turned to the locking position from the unlocking position through the operation of the actuating mechanism of the body part 24.

The second case body 16 integrally includes a substantially-rectangular bottom plate 30 which has a long edge in the left-right direction X and four side plates 31 erected from four sides of the bottom plate 30 towards a same direction. In the four side plates 31, two side plates 31 erected from a pair of long edges of the bottom plate 30 are called as long side plates 31A, and two side plates 31 erected from a pair of short edges of the bottom plate 30 are called as short side plates 31B. The long side plates 31A are longer than the short side plates 31B. In the two short side plates 31B, the short side plate 31B on the left side X1 is constructed between left ends of a pair of long side plates 31A, and the short side plate 31B on the right side X2 is constructed between right ends of a pair of long side plates 31A. Connecting parts between the bottom plate 30 and the side plates 31 and connecting parts between the side plates 31 can be in a shape of rounded corners as shown in FIG. 2, or a sharp shape with a substantially right angle.

The second case body 16 has a concave space 32 which is partitioned by the bottom plate 30 and the four side plates 31 and which is concave towards the bottom plate 30. The concave space 32 is exposed from a substantially-rectangular opening 36 partitioned by edges 31C of the four side plates 31 on the side opposed to the bottom plate 30. The operating part 7 is arranged on an outer side surface of the bottom plate 30 on the side opposed to an inner side surface facing the concave space 32.

On the left side surface of the short side plate 31B on the left side X1, two truckles 6 are arranged in a manner that the truckles 6 are away from each other in the front-rear direction Y. For the long side plate 31A on the upper side Z1 of the parallel two long side plates 31A in the up-down direction in FIG. 2, a clamping sheet 33 is arranged on the inner side surface facing the concave space 32. The clamping sheet 33 is formed into, for example, a plate shape made of metal, is disposed along and is fixed to the long side plate 31A on the upper side Z1. The clamping sheet 33 is formed with a through hole 33A through the clamping sheet 33 in the direction of the plate thickness.

The long side plate 31A on the lower side Z2 of the parallel two long side plates 31A in the up-down direction in FIG. 2 is connected with the long side plate 18A of the first case body 15 located on the rear side Y2 via hinges 34. Two hinges 34 are, for example, arranged in parallel in the left-right direction X. The first case body 15 and the second case body 16 are connected in a manner of turning relative to each other centering on the hinges 34. Under a state in FIG. 2, when the second case body 16 is turned towards the side of the first case body 15, the case part 2 can be in a closed state (see FIG. 1).

For the case part 2 in the closed state, the concave space 19 of the first case body 15 and the concave space 32 of the second case body 16 are integrated to form an internal space 3 of the case part 2. For the case part 2 in the closed state, the upper end part 18C of each side plate 18 of the first case body 15 is in a state of penetrating through the opening 36 of the second case body 16 and entering the concave space 32. At this moment, the edge 31C of each side plate 31 of the second case body 16 is in a state of being opposed to the stepped surface 18D of any side plate 18 throughout an overall region of long-edge directions of the side plates 18 and 31. A clearance between the edges 31C and the stepped surface 18D opposed to each other is filled with fillers 35 (see FIG. 1). The internal space 3 of the case part 2 in the closed state is sealed through the fillers 35 and the above fillers 22.

Under a condition that the locking part 25 of the unlocking mechanism 23 of the first case body 15 is located in the locking position, the locking part 25 is embedded into the through hole 33A of the clamping sheet 33 of the second case body 16 from the rear side Y2, and then clamped to the clamping sheet 33. Thus, since the closed state of the case part 2 is maintained, the user cannot open the case part 2. When the locking part 25 is turned to the unlocking position, the locking part 25 is separated from the through hole 33A. Thus, when the second case body 16 is turned for example away from the first case body 15, as shown in FIG. 2, the case part 2 can be opened. A suitcase with high air tightness can be used as the case part 2 with such a structure that can be opened and closed. Description is made below on the premise that the case part 2 is in the closed state.

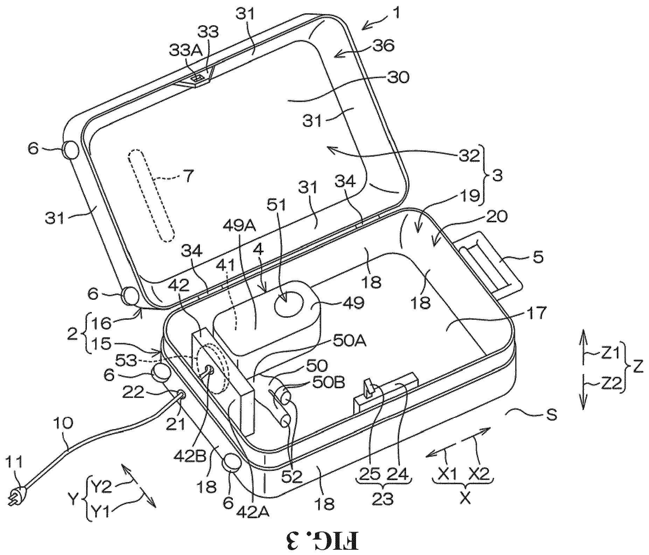

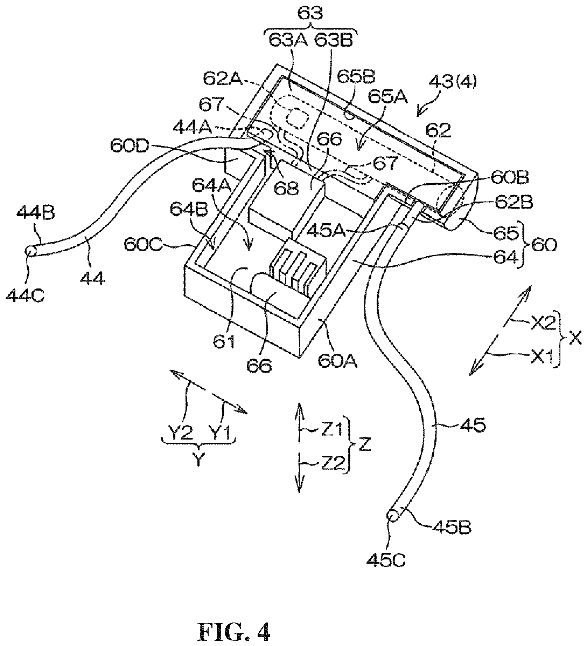

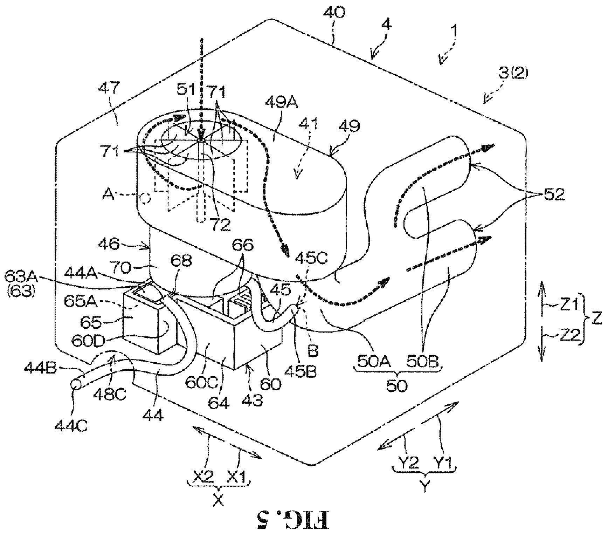

The purification unit 4 is disposed in the internal space 3, and is fixed to, for example, a position which is inclined to the left side X1 at the bottom plate 17 on the first case body 15. The article P as a purification object is accommodated in the internal space 3, and specifically, accommodated in the right side X2 region of the internal space 3 staggered with the the purification unit 4. The purification unit 4 is configured to purify the article P in the internal space 3 and includes: a cover 40, a flow path 41, a substrate case 42 (see FIG. 3 below), an ozone generating device 43 (see FIG. 4 below), a supply line 44 (see FIG. 4), a guide-in line 45 (see FIG. 4) and a fan 46 (see FIG. 5 below).

The cover 40 forms a housing of the purification unit 4, and is formed into a substantially-rectangular shape smaller than the case part 2. The cover 40 integrally includes a substantially-rectangular top wall 47 and four side walls 48 that extend from four sides of the top wall 47 towards the lower side Z2, and is fixed to the bottom plate 17 of the first case body 15. A round through hole 47A through the top wall 47 in the up-down direction Z is formed in a position of the top wall 47 slightly deviated to the right side X2 from the center. In the four side walls 48, the side wall 48 on the left side X1 is constructed between left ends of a pair of front and rear side walls 48, and the side wall 48 on the right side X2 is constructed between right ends of the pair of front and rear side walls 48. A notch 48A that extends from a lower end of the side wall 48 on the front side Y1 towards the upper side Z1 is formed in the side wall 48 on the front side Y1. A substantially-rectangular opening 48B through the side wall 48 along the front-rear direction Y is formed in the side wall 48 on the left side X1. The wire 10 penetrates through the opening 48B and extends into the cover 40.

By referring to FIG. 3 that illustrates a state that a cover 40 in FIG. 2 is removed, in correlation with the flow path 41, the purification unit 4 integrally includes a case-shaped first pipeline 49 that has a long edge in the left-right direction X and a tubular second pipeline 50 which is bent and extended to the front side Y1 after extending from a left end part of the first pipeline 49 towards the lower side Z2. Most of the first pipeline 49 and the second pipeline 50 are covered by the cover 40 from the upper side Z1 (see FIG. 2). The second pipeline 50 integrally includes a body part 50A that is bent after extending from the first pipeline 49 towards the lower side Z2, and two branch parts 50B which are branched up and down from a front end of the body part 50A. An internal space of the first pipeline 49 and an internal space of the second pipeline 50 are communicated with each other, and both internal spaces form the flow path 41. The two branch parts 50B are configured in a manner of being exposed from the notch 48A of the side wall 48 on the front side Y1 of the cove 40 to the front side Y1 (see FIG. 2).

The flow path 41 has a flow inlet 51 that forms one end of the flow path 41 and flow outlets 52 that form the other end of the flow path 41. The flow inlet 51 which is formed into a round shape penetrates through the top wall 49A of the first pipeline 49 in the up-down direction Z, is exposed from the through hole 47A of the top wall 47 of the cover 40 to the upper side Z1, and is in a state of communication with the internal space 3 (see FIG. 2). The flow outlets 52 which are formed into a round shape are respectively formed on the front ends of the two branch parts 50B and are exposed from the branch parts 50B to the front side Y1. That is, a plurality of flow outlets 52 are configured so that they have the same quantity as that of the branch parts 50B. Since the branch parts 50B are configured in a manner of being exposed from the side wall 48 on the front side Y1 of the cover 40 to the front side Y1, the flow outlets 52 are also configured in a manner of being exposed from the cover 40 to the front side Y1 and are in a state of communication with the internal space 3 (see FIG. 2).

The substrate case 42 is formed into a case shape which is flat in the left-right direction X, is configured in a position closer to the left side X1 than the first pipeline 49 and the second pipeline 50, and is covered by the cover 40 from the upper side Z1 together with the two pipelines (see FIG. 2). A main substrate (not shown) provided with a control part (not shown) composed of a microcomputer and the like is accommodated in the substrate case 42. The control part is electrically connected with the operating part 7, an executing mechanism of the unlocking mechanism 23, the ozone generating device 43 and the fan 46 through wires (not shown). Therefore, the control part can receive operation of the operating part 7 by the user, or can control actions of the executing mechanism to enable the locking part 25 to turn between the locking position and the unlocking position, or can control actions of the ozone generating device 43 and the fan 46.

A left side wall 42A of the substrate case 42 is formed with a through hole 42B through the left side wall 42A in the left-right direction X. The wire 10 (see FIG. 2), which penetrates through the opening 48B of the side wall 48 on the left side X1 of the cover 40 and extends into the cover 40, penetrates through the through hole 42B and is electrically connected with the main substrate within the substrate case 42. When the electric plug 11 is inserted into a socket (not shown), electric power from the socket is supplied to the operating part 7, the executing mechanism of the unlocking mechanism 23, the ozone generating device 43 and the fan 46 through the wire 10 and the main substrate. A drum-shaped reel 53 configured to wind the wire 10 can also be disposed in the substrate case 42. A force is exerted to the reel 53 by a force exerting member such as a spring towards a specified rotating direction. When the purification device 1 is moved, the reel 53 rotates towards the specified rotating direction to wind the wire 10 on an outer circumferential part of the reel 53 so that the wire 10 does not hinder movement of the purification device 1. When the purification device 1 is used after the movement, the wire 10 is pulled out.

By referring to a perspective diagram (FIG. 4) illustrating the ozone generating device 43, the ozone generating device 43 includes: a device shell 60, a discharging substrate 61, a discharging tube 62 and a lid 63. The device shell 60 is formed into a case shape of which the entire region of an upper surface is open.

The device shell 60 integrally includes a left part 64 and a right part 65 which is connected to the left part 64 from the right side X2 and formed to be wider in the front-rear direction Y than the left part 64. The overall device shell 60 is formed into a substantial T shape when observed from the upper side Z1. Therefore, when observed from the upper side Z1, a front side wall 60A of the device shell 60 is bent into a crank shape in a manner of having a front extending part 60B that extends in a middle of the left-right direction X towards the front side Y1; and a rear side wall 60C of the device shell 60 is bent into a crank shape in a manner of having a rear extending part 60D that extends in the middle of the left-right direction X to the rear side Y2. An opening 64B that exposes an overall region of an internal space 64A of the left part 64 to the upper side Z1 is formed in an upper surface of the left part 64, and an opening 65B that exposes an overall region of an internal space 65A of the right part 65 to the upper side Z1 is formed in an upper surface of the right part 65. The internal space 64A and the internal space 65A are communicated with each other, and the opening 64B and the opening 65B are communicated with each other.

The discharging substrate 61 is accommodated in the internal space 64A of the left part 64, and is exposed out of the device shell 60 from the opening 64B. On the discharging substrate 61, elements 66 such as a capacitor, a transistor and the like are installed from the upper side Z1. The discharging substrate 61 is electrically connected to the above main substrate. The discharging tube 62 is a metal round tube extending in the front-rear direction Y, and is accommodated in the internal space 65A of the right part 65. An inlet 62A communicated to an interior of the discharging tube 62 is formed in an outer circumferential surface at a lower side of a rear end part of the discharging tube 62, and a tubular outlet member 62B protruded from the discharging tube 62 towards the left side X1 is integrally arranged in a front end part of the discharging tube 62. To prevent foreign matter such as dust and the like from entering the discharging tube 62, a filtering member such as a sponge and the like can also be arranged on the inlet 62A. The outlet member 62B is in a state of penetrating through the front extending part 60B and protruding further towards the left side X1 than the front extending part 60B. The discharging substrate 61 and the discharging tube 62 are in a state of being electrically connected via wrings 67.

The lid 63 integrally includes: a transverse plate part 63A having approximately the same size as the opening 65B of the right part 65 and having a long edge in the front-rear direction Y, and a longitudinal plate part 63B that extends from a middle part in the front-rear direction Y at a left edge of the transverse plate part 63A towards the lower side Z2. A plate thickness of the transverse plate part 63A is in the up-down direction Z, and a plate thickness of the longitudinal plate part 63B is in the left-right direction X. The transverse plate part 63A blocks almost the entire region of the opening 65B from the upper side Z1. The longitudinal plate part 63B is configured between the front extending part 60B and the rear extending part 60D of the device shell 60 to separate the internal space 64A from the internal space 65A. A rear end part of the left edge of the transverse plate part 63A is separated with the rear extending part 60D from the right side X2 by a small clearance 68 and opposed to the rear extending part 60D. The internal space 65A is exposed out of the device shell 60 via the clearance 68.

The supply line 44 and the guide-in line 45 are flexible tubes. For the supply line 44, one end part 44A is inserted into the clearance 68 and disposed near the inlet 62A of the discharging tube 62 located in the internal space 65A of the right part 65 of the device shell 60, and the other end part 44B is formed with an intake port 44C. For the guide-in line 45, one end part 45A is connected to the outlet member 62B of the discharging tube 62, and the other end part 45B is formed with an output port 45C.

By referring to a perspective diagram (FIG. 5) illustrating the purification unit 4, the ozone generating device 43 is disposed on the lower side Z2 of the first pipeline 49 in a state of being covered by the cover 40 from the upper side Z1. A substantially-semicircular notch 48C configured to cut a local lower edge of the side wall 48 is formed in the lower end part of the side wall 48 of the cover 40 on the rear side Y2. Since the other end part 44B of the supply line 44 penetrates through the notch 48C and is pulled out of the cover 40, the intake port 44C of the other end part 44B is exposed out of the cover 40 to the internal space 3 of the case part 2. One end part 45A of the guide-in line 45 (see FIG. 4) is connected to the discharging tube 62 of the ozone generating device 43. The overall guide-in line 45 is configured in the cover 40. For the guide-in line 45, the other end part 45B is connected to the body part 50A of the second pipeline 50 from the rear side Y2, and the output port 45C of the other end part 45B is communicated to the flow path 41 of the second pipeline 50. In this way, the guide-in line 45 connects the ozone generating device 43 with the flow path 41.

A multi-blade fan can be taken as an example of the fan 46. The fan 46 includes: a motor 70 disposed between the ozone generating device 43 and the first pipeline 49, and rotation blades 71 disposed in the internal space of the first pipeline 49 (that is, the flow path 41). The motor 70 has an output shaft 72 protruded towards the upper side Z1 and disposed in the internal space of the first pipeline 49. A plurality of rotation blades 71 are disposed and fixed to the output shaft 72 in a manner of expanding radially from the output shaft 72. The rotation blades 71 and the output shaft 72 are in a state of being exposed from the flow inlet 51 of the flow path 41 towards the upper side Z1. When purification operation is started and the fan 46 is driven, the output shaft 72 rotates with the rotation blades 71. A rotating direction of the rotation blades 71 is, for example, a clockwise rotation direction when observed from the upper side Z1.

Through the rotation of the rotation blades 71 under the driving of the fan 46, air in the internal space 3 of the case part 2, as shown by a thick dotted arrow, penetrates through the flow inlet 51 which is exposed from the top wall 47 of the cover 40 and flows into the flow path 41 from the upper side Z1. The air which flows into the flow path 41 from the flow inlet 51 flows from the flow inlet 51 in the flow path 41 to the flow outlet 52 through the rotation of the rotation blades 71. Specifically, after the air which flows in the flow path 41 as shown by the thick dotted arrow rotates with the rotation of the rotation blades 71, the air flows into the second pipeline 50 after advancing in the first pipeline 49 towards the left side X1, and flows out of any flow outlet 52 into the internal space 3. Thus, an air flow that circulates between the flow path 41 and the internal space 3 is produced (see the thick dotted arrow in FIG. 2).

In the purification operation, the ozone generating device 43 exerts high voltage to the discharging tube 62 from the discharging substrate 61, and produces discharge in the discharging tube 62 (see FIG. 4). The supply line 44 takes the air in the internal space 3 from the intake port 44C exposed to the internal space 3. The air is supplied from one end part 44A of the supply line 44 to the internal space 65A in the right part 65 of the device shell 60 of the ozone generating device 43. The air supplied to the internal space 65A flows into the discharging tube 62 from the inlet 62A of the discharging tube 62 accommodated in the internal space 65A, and flows to the outlet member 62B of the discharging tube 62 (see FIG. 4). At this moment, through the discharge generated in the discharging tube 62 to which high voltage is exerted, the air which flows in the discharging tube 62 generates the ozone. In this way, after the ozone generated by the ozone generating device 43 flows through the guide-in line 45 from the outlet member 62B, the ozone is guided into the flow path 41 from the output port 45C located in the other end part 45B of the guide-in line 45. At this moment, the ozone in the guide-in line 45 is pulled by the air flow in the flow path 41 and is actively guided to the flow path 41. The ozone that reaches the flow path 41 flows from the flow outlet 52 of the flow path 41 to the internal space 3 along with the air that flows in the flow path 41, and then is pouring on the article P in the internal space 3. Thus, the article P is purified by the ozone.

Since the purification of the article P by the ozone can be promoted with rise of the concentration of the ozone in the internal space 3 of the case part 2, the purification performance of the purification device 1 can be enhanced. Especially, since the purification operation of the purification device 1 is assumed that the article P accommodated in the internal space 3 is purified by the ozone for a short time of about 30 minutes to 1 hour, it is preferable the ozone concentration is high. It should be noted that the internal space 3 in the purification operation, as mentioned above, is sealed by the fillers 22 and the fillers 35, and the locking part 25 of the unlocking mechanism 23 is located in the locking position to be kept in a state that the case part 2 is closed, thereby preventing the ozone from being leaked out of the case part 2. It should be noted that description is made below on the premise of performing the purification operation for 30 minutes, but the purification operation under this situation is implemented to eliminate, for example, odor noticed by an user before going out.

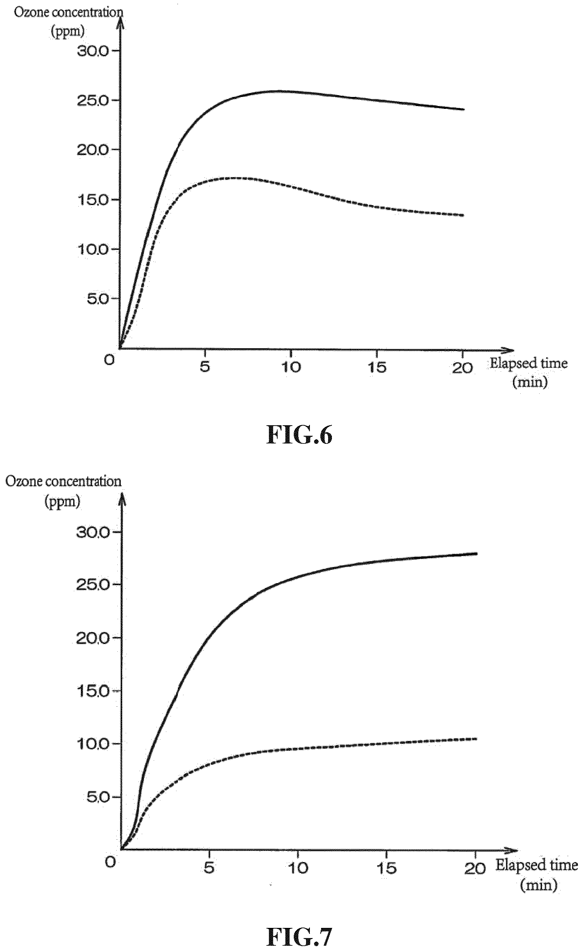

FIG. 6 is a curve chart illustrating a relationship between a supply line 44 connected to the ozone generating device 43 and a time-dependent change of the ozone concentration produced by the ozone generating device 43. In the curve chart of FIG. 6, a transverse axis represents time elapsed from the beginning of the purification operation, and a longitudinal axis represents the ozone concentration in the internal space 3 in the purification operation. The unit of the elapsed time is minute, and the unit of the ozone concentration is ppm.

For the purification unit 4, since high voltage is generated on the ozone generating device 43, at least the ozone generating device 43 needs to be covered by the cover 40 in such a manner that the user cannot contact the ozone generating device 43. In addition, since the space for accommodating the article P in the internal space 3 becomes small when the ozone generating device 43 is disposed outside the cover 40, it is better to configure the ozone generating device 43 in the cover 40.

Herein, a first comparison example in which the intake port 44C (see FIG. 5) of the supply line 44 is configured in the cover 40 and not exposed to the internal space 3 is discussed. In the first comparison example, structures except the position of the intake port 44C are the same as those of the present embodiment. In the case of the first comparison example, since it is difficult to actively take the air outside the cover 40 from the intake port 44C located in the cover 40 without the air flow and to supply the air to the ozone generating device 43, the ozone concentration in the purification operation is only increased to about 15 ppm as shown by the thick dash line in FIG. 6.

In another aspect, in the present embodiment, since the intake port 44C is exposed to the internal space 3 of the case part 2 outside the cover 40 as mentioned above, the intake port 44C is exposed to the air flow that circulates between the flow path 41 and the internal space 3. Thus, the supply line 44 forcibly takes the air that flows in the internal space 3 from the intake port 44C, thereby promoting the air supply to the ozone generating device 43. Therefore, since the ozone generating device 43 can produce more ozone, in the present embodiment, the ozone concentration in the internal space 3 in the purification operation can be increased to about 25 ppm as shown by the thick solid line in FIG. 6, thereby realizing the improvement of the purification performance. It should be noted that, from a perspective of aesthetics, the intake port 44C can be disposed in an inconspicuous position in the internal space 3 as much as possible, e.g., between the cover 40 and the long side plate 18A of the first case body 15 on the side of the hinge 34 (see FIG. 2).

FIG. 7 is a curve chart illustrating a relationship between a guide-in line 45 connected to the ozone generating device 43 and a time-dependent change of the ozone concentration produced by the ozone generating device 43. In the curve chart of FIG. 7, a transverse axis represents time elapsed from the beginning of the purification operation, and a longitudinal axis represents the ozone concentration in the internal space 3 in the purification operation. The unit of the elapsed time is minute, and the unit of the ozone concentration is ppm.

A second comparison example in which the other end part 45B of the guide-in line 45 is connected to a region A around the rotation blades 71 of the flow path 41 (see FIG. 5) is discussed. In the second comparison example, structures except the position of the other end part 45B are the same as those of the present embodiment. In the case of the second comparison example, since part of the ozone guided into the region A of the flow path 41 from the other end part 45B is dispersed by the rotating rotation blades 71 until flowing out to the internal space 3, the ozone concentration in the purification operation is only increased to about 10 ppm as shown by the thick dash line in FIG. 7.

In another aspect, in the present embodiment, since the other end part 45B of the guide-in line 45 is connected to the body part 50A of the second pipeline 50 as mentioned above, the other end part 45B is connected to a region B of the flow path 41, closer to the side of the flow outlet 52 (i.e., a downstream side of the air flow of the flow path 41) than the rotation blades 71 (see FIG. 5). Thus, the ozone guided into the flow path 41 can flow out of the flow outlet 52 to the internal space 3 through the guide-in line 45, and is not dispersed by the rotating rotation blades 71. Thus, in the present embodiment, the ozone concentration in the internal space 3 in the purification operation can be increased to about 25 ppm as shown by the thick solid line in FIG. 7, thereby realizing further improvement of the purification performance.

In this way, it can be seen through comparison of the first comparison example and the second comparison example with the present embodiment that, even if the same ozone generating device 43 is used, the ozone concentration in the purification operation can be increased rapidly by trying to adjust the positions of the intake port 44C of the supply line 44 and the other end part 45B of the guide-in line 45. It should be noted that in a later stage of the above purification operation of 30 minutes, for example, after 20 minutes from the beginning of the purification operation, the ozone generating device 43 is stopped, but the fan 46 continues to rotate. Thus, the ozone in the case part 2 not only dissipates naturally, but also is forcibly dispersed by the rotating rotation blades 71 of the fan 46. Therefore, after the purification operation is ended, the ozone hardly exists in the case part 2. After the purification operation is ended, the locking part 25 of the unlocking mechanism 23 is switched from the locking position to the unlocking position through the above control part (not shown). Therefore, the user can open the case part 2 to take out the purified article P.

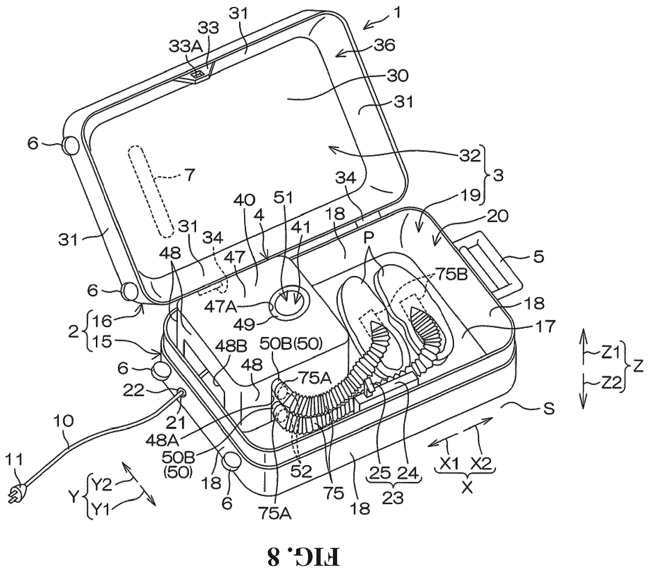

FIG. 8 is a perspective diagram illustrating a purification device 1 which is put down and opened and in which the article P is placed. A plurality of articles P like a pair of shoes in FIG. 8 may be place in the purification device. Under this situation, since a plurality of flow outlets 52 that enable the ozone to flow out of the flow path 41 are formed, the ozone is pouring on the plurality of articles P from the respective flow outlets 52. As a result, the articles P can be purified simultaneously.

However, under a state that the positions of the flow outlets 52 are fixed, considering sizes and shapes of the articles P, it is possible that the articles P cannot be disposed in the position of the flow outlets 52 in the internal space 3 as shown in FIG. 8. Therefore, the purification device 1 includes hoses 75 connected to two flow outlets 52 of the flow path 41 respectively. In FIG. 8, two hoses 75 are provided. Each hose 75 has the same flexibility and stretchability as those of a corrugated hose. One end part 75A of each hose 75 is externally embedded on a branch part 50B of the second pipeline 50 formed with the flow outlet 52. The hoses 75 can be bent or stretched so that the other end parts 75B of the hoses 75 face the articles P and the ozone from the flow outlets 52 is poured directly on the articles P from the other end parts 75B. Especially, under a condition that the articles P are shoes and bags which have deeper interiors, the other end parts 75B are inserted into the articles P so that the ozone is poured on the interiors of the articles P. It should be noted that the quantity of the flow outlets 52 is not limited to two, and may be more than two.

The present invention is not limited to above described embodiments, and various changes can be made within a scope claimed in claims.

For example, the above purification device 1 has the handle 5 and the truckles 6 and thus can be moved. However, if move is not considered, the purification device 1 can be, for example, fixed to a specified indoor position.

In addition, one end part 44A of the supply line 44 is inserted into the clearance 68 of the device shell 60 and thus is disposed in the internal space 65A of the right part 65 of the device shell 60 (see FIG. 4). Instead, one end part 44A can penetrate through the right part 65 and is directly connected to the inlet 62A of the discharging tube 62 in the internal space 65A. Under this situation, the clearance 68 can be blocked.

## Claims

1. A purification device, comprising:
a case part having an internal space for accommodating an article; and a purification unit disposed in the internal space and used to purify the article in the internal space, wherein
the purification unit comprises:
a flow path having a flow inlet and a flow outlet which are in communication with the internal space and enabling air to flow from the flow inlet to the flow outlet;
a fan having a rotation blade disposed on the flow path, wherein the rotation of the rotation blade enables the air in the internal space to flow in the flow path from the flow inlet and to flow out to the internal space from the flow outlet, so that the air circulates between the flow path and the internal space;
an ozone generating device generating ozone from air;
a cover at least covering the ozone generating device;
a supply line having an intake port exposed outside of the cover to the internal space, taking the air from the internal space through the intake port and supplying the air to the ozone generating device; and
a guide-in line connecting the ozone generating device and the flow path and guiding the ozone generated by the ozone generating device into the flow path.

2. The purification device according to claim 1, wherein
the guide-in line is connected to a region of the flow path closer to a side of the flow outlet than the rotation blade.

3. The purification device according to claim 1 or 2, wherein
the purification device comprises at least one of a handle and truckles arranged on the case part.

4. The purification device according to any one of claims 1-3, wherein
the purification device is provided with a plurality of flow outlets; and
the purification device comprises a hose connected to the flow outlets and having flexibility and stretchability.
